Europäisches Patentamt

European Patent Office

Office européen des brevets

(19)

(11) Veröffentlichungsnummer: **0 363 671 B1**

(12) **EUROPÄISCHE PATENTSCHRIFT**

(45) Veröffentlichungstag der Patentschrift: **26.01.94**

(51) Int. Cl.5: **A61K 37/64**

(21) Anmeldenummer: **89117050.8**

(22) Anmeldetag: **14.09.89**

(54) Verwendung von ACE-Inhibitoren gegen Neointimabildung nach Gefässverletzung.

(30) Priorität: **14.09.88 CH 3421/88**

(43) Veröffentlichungstag der Anmeldung:
**18.04.90 Patentblatt 90/16**

(45) Bekanntmachung des Hinweises auf die
Patenterteilung:
**26.01.94 Patentblatt 94/04**

(84) Benannte Vertragsstaaten:
**AT BE CH DE ES FR GB GR IT LI LU NL SE**

(56) Entgegenhaltungen:

CHEMICAL ABSTRACTS, Band 96, Nr. 25, 21.
Juni 1982, Seite 46, Zusammenfassung Nr.
210615n, Columbus, Ohio, US; H. YOSHIMO-
TO et al.: "A study on antihypertensive and
antisclerotic effects of captopril on the retinal arteriole in SHRSP", & NIPPON GANKA
GAKKAI ZASSHI 1982, 86(2), 227-38

(73) Patentinhaber: **F. HOFFMANN-LA ROCHE AG**
**Postfach 3255**
**CH-4002 Basel(CH)**

(72) Erfinder: **Baumgartner, Hans Rudolf, Prof. Dr.**
**Bodenweg 38**
**CH-4144 Arlesheim(CH)**
Erfinder: **Clozel, Jean-Paul, Dr.**
**11 Rue Oberlin**
**F-68300 St. Louis(FR)**
Erfinder: **Hefti, Fridolin, Dr.**
**Strengigässli 18**
**CH-4123 Allschwil(CH)**
Erfinder: **Hosang, Markus, Dr.**
**Burgfeldermattweg 59**
**CH-4123 Allschwil(CH)**
Erfinder: **Kuhn, Herbert, Dr.**
**Wydenweg**
**CH-4112 Flüh(CH)**
Erfinder: **Müller, Rita**
**Eulerstrasse 24**
**CH-4051 Basel(CH)**
Erfinder: **Powell, Jerry, Dr.**
**Hirzbrunnenschanze 37**
**CH-4058 Basel(CH)**

EP 0 363 671 B1

CHEMICAL ABSTRACTS, Band 99, Nr. 7, 15. August 1983, Seite 37, Zusammenfassung Nr. 47727b, Columbus, Ohio, US; K. YAMAGA-MI et al.: "The antihypertensive and antisclerotic effects of captopril on the retinal arteriole. Comparison of administration at 8 and 46 weeks of age in rats" & NIPPON GANKA KIYO 1983, 34(2), 290-7

SCIENCE, Band 245, Nr. 4914, 14. Juli 1989, Seiten 186-188; J.S. POWELL et al.: "Inhibitors of angiotensin-converting enzyme prevent myointimal proliferation after vascular injury"

AM. J. PATHOL., Band 117, Nr. 3, 1984, Seiten 360-371; C. LIMAS et al.: "Comparitve effects of hydralazine and captopril on the cardiovascular changes in spontaneously hypertensive rats"

JOURNAL OF CARDIOVASCULAR PHARMACOLOGY, Band 16(Suppl. 4), 1990, Seiten S42-S49, Raven Press Ltd, New York, US; J.S. POWELL et al.: "The proliferative response to vascular injury is suppressed by angiotensin-converting enzyme inhibition"

(74) Vertreter: Lederer, Franz, Dr. et al
Lederer, Keller & Riederer
Patentanwälte
Lucile-Grahn-Strasse 22
D-81675 München (DE)

**Beschreibung**

Aus Chemical Abstracts **96**, (25), 21. Juni, 1982, Seite 46, Zusammenfassung Nr.216015n und Chemical Abstracts **99**, (7), 15. August, 1983, Seite 37, Zusammenfassung Nr. 47727b ist bekannt, dass Captopril an spontan hypertonen Ratten den Blutdruck senkt und antisklerotisch wirkt, was auf die den peripheren Gefässwiderstand reduzierende Wirkung von Captopril zurückgeführt wird. In Am. J. Pathol. **117**, (3), 1984, Seiten 360-371 wird beschrieben, dass Captopril bei spontan hypertonen Ratten nicht nur eine Blutdrucksenkung, sondern auch eine Abnahme der Dicke der Media der Gefässwand innerhalb der Aorta bewirkt.

Im Rahmen der vorliegenden Erfindung wurde festgestellt, dass ACE-Inhibitoren die Bildung von Neointima und die Reduktion des Gefässlumens durch Gefässwandverdickung nach einer Gefässverletzung unterdrücken.

Die Vermehrung von glattem Muskelgewebe durch Wucherung oder Sprossung (Proliferation) und die Bildung einer extrazellulären Matrix sind die Hauptprozesse, die zu einer Verdickung der Gefässwand und damit zum Gefässverschluss bei der Arteriosklerose (einschliesslich Atherosklerose) oder nach Gefässchirurgie oder gewaltsamer Gefässöffnung nach Gefäss -stenose oder -verschluss, z.B. nach einem Herzinfarkt, führen. Diese Prozesse, die beim Gefässwachstum während der Entwicklung und als Antwort auf eine Gefässdehnung oder -verletzung vor sich gehen, sind normalerweise harmlos. Wenn sie jedoch überschiessen, führen sie zur Einengung von Arterien und damit zu erhöhter Morbidität, d.h. Krankheit im weitesten Sinn.

Die Proliferation von glatten Muskelzellen in der Gefässwand vollzieht sich in der Frühphase der Heilung nach einer Verletzung des Endothels , d.h. der das Gefäss innen auskleidenden Zellschicht. So ist zum Beispiel in der Halsschlagader (Arteria carotis) einer Ratte der Uebergang der Zellen aus einem ruhenden Zustand in einen Wachstumszustand innerhalb von 24 Stunden im wesentlichen abgeschlossen. Die Proliferation wird durch fortgesetztes Wachstum und Kernteilung (Mitosis) der Nachkommenschaft dieser ursprünglich stimulierten Zellen verstärkt (Laboratory Investigation, 49, 327-333 (1983)). Die Wachstumsfaktoren sowie andere Mechanismen, durch die bislang ruhige glatte Muskelzellen zur Kernteilung veranlasst werden, sind nicht bzw. nur ungenügend bekannt. Bekannt ist nur, dass das Wachstum von glatten Muskelzellen in vitro von Wachstumsfaktoren aus den Blutplättchen verstärkt wird, wobei aber deren Rolle in vivo noch nicht geklärt ist (The new England Journal of Medicine, 314, 488-500, (1986)).

Nach einer Verletzung, im weitesten Sinne nach jeder Störung des Gefässendothels oder der Gefässwand, werden die glatten Muskelzellen der Media der Gefässwand dazu veranlasst, in den Wachstumszustand einzutreten, d.h. zu proliferieren und extrazelluläre Matrix herzustellen. Zusätzlich zur Synthese von DNA und zur Mitose wandert ein Teil der glatten Muskelzellen in die Intima der Gefässwand und proliferiert dort. Einige der in die Intima eingewanderten glatten Muskelzellen stellen ebenfalls extrazelluläre Matrix her und tragen dabei wesentlich zu der Verdickung der Gefässwand bei, wie sie nach einer Gefässverletzung beobachtet wird. Vereinfacht gesehen besteht der Ablauf der Verdickung der Intima einer verletzten Arterie in einer anfänglichen Proliferation der glatten Muskelzellen in der Media, der eine Einwanderung solcher Zellen von der Media in die Intima folgt. Zusätzlich proliferieren glatte Muskelzellen in der Intima und produzieren extrazelluläre Matrix. Letztendlich können intimale Proliferation und Matrixbildung zu einer Einschränkung des Gefässquerschnitts in einem solchen Ausmass führen, dass Morbidität eintritt (The New England Journal of Medicine, 318, 1734-1737, (1988), and Journal of the American College of Cardiology, 6, 369-375, (1985)).

Die Vorgänge, die zur Bildung von Neointima führen, beruhen auf mehreren grundlegenden Mechanismen:

1. Die Proliferation der glatten Muskelzellen vergrössert ihre Anzahl (Hyperplasie) in Media und Intima.
2. Es findet Migration von glatten Muskelzellen von der Media in die Intima statt.
3. Extrazelluläre Matrix wird gebildet.

Die Summe dieser hauptsächlich in der Intima stattfindenden Prozesse führt zur Bildung von neointimalem Gewebe und damit zu einer Beeinträchtigung des Blutflusses im Gefäss.

Wirkstoffe, die diese Prozesse verhindern oder stark einschränken, sind bei einer Reihe von pathologischen Zuständen von therapeutischem Wert. Es ist bekannt, dass verschiedene Wirkstoffe die Proliferation von glatten Muskelzellen in vitro hemmen, jedoch ist es bisher nur für Heparin eindeutig möglich gewesen, eine Hemmung der Proliferation glatter Muskelzellen und der Bildung der Neointima auch in Tiermodellen nachzuweisen (Nature, 265, 625-626 (1977)). Allerdings liegt die Plasmakonzentration, die für das Erzielen dieser Effekte in Tieren nötig ist, 3 bis 4 mal höher als die für den Menschen therapeutisch übliche Konzentration. Im weiteren kann Heparin nur intravenös verabreicht werden, was die Verabreichungsdauer stark limitiert.

Wie bereits oben erwähnt, wurde im Rahmen der vorliegenden Erfindung festgestellt, dass ACE-Inhibitoren die Bildung von Neointima und die Reduktion des Gefässlumens durch Gefässwandverdickung nach einer Gefässverletzung unterdrücken, und zwar in Konzentrationen, welche in der gleichen Grössenordnung liegen, wie diejenigen, die bei der Behandlung von Bluthochdruck mit ACE-Inhibitoren üblich sind.

Es ist zu erwarten, dass die erfindungsgemässe Unterdrückung der Bildung von Neointima und die damit verbundene Verhinderung der Reduktion des Gefässlumens durch Gefässwandverdickung nach einer Gefässverletzung mittels ACE-Inhibitoren bei einer Reihe von klinischen Situationen von therapeutischem Wert sein wird. Dazu gehören die Verhinderung einer Restenosierung nach perkutaner transluminaler Angioplastie oder Gefässchirurgie sowie die Behandlung bzw. Verhinderung von Neointimabildung und von Gefässwandverdickung bei Arteriosklerose und diabetischer Angiopathie.

Gegenstand der vorliegenden Erfindung ist die Verwendung von ACE-Inhibitoren zur Verhinderung der Proliferation glatter Muskelzellen in der Intima und/oder der Media, der Migration glatter Muskelzellen von der Media in die Intima und der Bildung extrazellulärer Matrix sowie zur Behandlung und Verhinderung von Neointimabildung, jeweils nach Gefässverletzung, insbesondere von einer physikalisch, chemisch oder chirurgisch bedingten Verletzung, wie von arterieller Restenosierung nach Angioplastie oder Gefässwandverdickung nach Gefässchirurgie in Arterien und Venen. Gegenstand der vorliegenden Erfindung ist auch die Verwendung von ACE-Inhibitoren zur Prophylaxe von Arteriosklerose und diabetischen Angiopathien.

Das Angiotensin-konvertierende Enzym ist für die Spaltung des kreislaufinaktiven Angiotensin I zu Angiotensin II verantwortlich. Die hauptsächlichen Wirkungen von Angiotensin II sind bekannt. Angiotensin II ist ein potenter Konstriktor von glatter Muskulatur, insbesondere von Gefässmuskulatur. Es stimuliert die Freisetzung von Noradrenalin und hemmt dessen Wiederaufnahme in Nervenendigungen, wodurch es zusätzlich vasokonstriktorisch wirkt. Es beeinflusst auch die Prostaglandinfreisetzung in einer solchen Weise, dass es ebenfalls konstriktorisch wirkt. Angiotensin II fördert aber auch die Gefässneubildung (Angiogenese). Auf den drei erstgenannten Hauptwirkungen beruht die Verwendung von ACE-Inhibitoren als Antihypertensiva, nämlich durch die Senkung des Plasmaspiegels von Angiotensin II und einer sich daraus ergebenden Abnahme des gesamten Gefässwiderstandes (Journal of Clinical Investigation 79, 1-6, (1987)).

Für den Zweck der vorliegenden Erfindung geeignete ACE-Inhibitoren sind Alacepril, Benazepril, Captopril, Cilazapril, Delapril, Enalapril, Enalaprilat, Fosinopril, Lisinopril, Perindopril, Quinapril, Ramipril, Spirapril, Zofenopril und MC 838 [Calciumsalz von (R-(R,S))-1-(3-((2-((Cyclohexylcarbonyl)amino)-1-oxopropyl)thio)-2-methyl-1-oxopropyl)-L-prolin] sowie Analoga dieser Verbindungen wie sie in den Europäischen Patentpublikationen EPA 7.477, 12.401, 50.800, 51.391, 53.902, 65.301, 72.352, 94.095, 172.552, 211.220 und 271.795, den US Patentschriften 4.105.776 und 4.316.906, der Britischen Patentschrift 2.102.412 sowie Tetrahedron Letters, 23, 1677 - 1680 (1982) beschrieben sind. Bevorzugte ACE-Inhibitoren sind die in den genannten Publikationen als bevorzugt hervorgehobenen Verbindungen. Ganz besonders bevorzugte ACE-Inhibitoren sind die weiter oben spezifisch genannten Verbindungen. Der bevorzugteste ACE-Inhibitor ist das Cilazapril.

Zur Verhinderung der traumainduzierten Neointimabildung werden die ACE-Inhibitoren, besonders bevorzugt das Cilazapril, systemisch, vorzugsweise enteral, besonders bevorzugt oral, verabreicht. Die Dosis variiert gemäss den Bedürfnissen des einzelnen Patienten, wie sie vom behandelnden Arzt bestimmt worden ist. Für das besonders bevorzugte Cilazapril dürfte im allgemeinen eine tägliche Dosis von etwa 0,5 bis 10 mg, vorzugsweise 2,5 oder 5 mg oral pro Tag verwendet werden. Die Dosis von 2,5 oder 5 mg pro Tag ist diejenige, die der therapeutisch bevorzugt eingesetzten Dosis für die Behandlung des Bluthochdrucks mit Cilazapril entspricht. Zur Verhinderung einer Restenosierung nach Angioplastie sollte der ACE-Inhibitor vorzugsweise vor der Intervention gegeben werden und danach so lange, bis das Risiko einer Restenosierung vernachlässigbar geworden ist, d.h. etwa ein Jahr. Die anderen ACE-Inhibitoren können in Uebereinstimmung mit der jeweiligen Dosierungsvorschrift, im einzelnen festgelegt durch den behandelnden Arzt, verabreicht werden.

In diesem Zusammenhang soll auch noch darauf hingewiesen werden, dass wegen der Möglichkeit der oralen Verabreichung der ACE-Inhibitoren eine Behandlungsdauer zeitlich nicht begrenzt ist, was für die erfindungsgemässe Verwendung wesentlich ist.

Als Verabreichungsform kommen die für die systemische Verabreichung üblichen festen oder flüssigen Darreichungsformen in Betracht, z.B. Suppositorien oder, als feste orale Darreichungsformen, Kapseln, Tabletten, Lacktabletten, Dragées, Pillen, Puder, Granulate und dergleichen, als flüssige orale Darreichungsformen Lösungen, Sirupe, Suspensionen, Elixiere und dergleichen und als parenterale Darreichungsformen Infusions- oder Injektionslösungen, die intravenös oder intramuskulär injiziert werden können.

Es liegt im Bereich der vorliegenden Erfindung, die ACE-Inhibitoren, vorzugsweise das Cilazapril, in jeder für die Verabreichung geeigneten Menge in die enterale oder orale Darreichungsform einzuarbeiten. Es ist jedoch bevorzugt, Präparate herzustellen, die pro Dosierungseinheit den Wirkstoff in einer Menge von

EP 0 363 671 B1

etwa 0,5 - 10 mg, vorzugsweise von etwa 2,5 oder 5 mg, enthalten. Besonders bevorzugt ist die Herstellung von Kapseln und Lacktabletten.

Die Herstellung der oben genannten Gebrauchsformen kann in üblicher Weise, z.B. anhand der nachstehenden Beispiele für Cilazapril erfolgen.

**Beispiel 1**

```
        Tabletten à 0,5 mg Cilapril



        Zusammensetzung            mg / Tablette


        1. Cilazapril (Monohydrat)    0,522 (entspr. 0,5 mg Base)
        2. Lactose pulv.            82,028
        3. Maisstärke               39,000
        4. Hydroxypropylmethylcellulose  5,200
        5. Talk                      1,950
        6. Natriumstearylfumarat     1,300
                                     ------
        Gewicht pro Tablettenkern   130,000



        Zusammensetzung des Lacks:   mg / Lacktablette


        7. Hydroxypropylmethylcellulose  1,50
        8. Talk                      0,75
        9. Titandioxid               0,75
                                     ----
                                     3,00


        Totalgewicht pro Lacktablette  133.00 mg
```

Der Wirkstoff wird sukzessive mit einer Mischung aus Lactose und Maisstärke homogen vermischt, gesiebt und mit einer wässrigen Hydroxypropylmethylcellulose-Lösung befeuchtet, granuliert und getrocknet. Dem getrockneten Granulat wird Talk und Natriumstearylfumarat zugemischt und aus der pressfertigen Mischung werden Tabletten geeigneter Grösse hergestellt. Die Tablettenkerne werden mit einer wässrigen Suspension der Komponenten 7-9 nach einem geeigneten Lackierverfahren lackiert.

5

**Beispiel 2**

Lacktabletten à 10 mg Cilapril

| Zusammensetzung | mg / Tablette |
|---|---|
| 1. Cilazapril (Monohydrat) | 10,44 (entspr. 10 mg Base) |
| 2. Lactose pulvis | 243,56 |
| 3. Maisstärke | 120,00 |
| 4. Hydroxypropylmethylcellulose | 16,00 |
| 5. Talk | 6,00 |
| 6. Natriumstearylfumarat | 4,00 |
|  | ------- |
| Gewicht pro Tablettenkern | 400,000 |

| Zusammensetzung des Lacks: | mg / Lacktablette |
|---|---|
| 7. Hydroxypropylmethylcellulose | 5,00 |
| 8. Talk | 2,50 |
| 9. Titandioxid | 0,50 |
| 10. Eisenoxid rot | 2,00 |
| Totalgewicht pro Lacktablette | 410,00 mg |

Der Wirkstoff Cilazapril wird mit den Komponenten 2-4 homogen vermischt, mit einer wässrigen Lösung von 4 befeuchtet, granuliert und getrocknet. Das getrocknete Granulat wird mit Talk und Natriumstearylfumarat homogen vermischt und zu Tablettenkernen geeigneter Form verpresst. Die Tablettenkerne werden mit einer wässrigen Suspension der Komponenten 7 - 10 nach einem geeigneten Lackierverfahren lackiert.

**Beispiel 3**

| Hartgelatinekapseln à 5 mg Cilazapril | |
|---|---|
| Zusammensetzung | mg / Kapsel |
| 1. Cilazapril (Monohydrat) | 5,22 (entspr. 5,0 mg Base) |
| 2. Lactose pulv | 93,94 |
| 3. Lactose krist. | 110,00 |
| 4. Stärke STA-RX 1500 | 48,00 |
| 5. Talk | 22,00 |
| 6. Magnesiumstearat | 0,84 |
| Gewicht pro Kapselfüllung | 280,00 mg |

Der Wirkstoff Cilazapril wird mit Lactose pulv. sukzessive homogen vermischt und gesiebt (I). Die Komponenten 3 - 5 werden homogen gemischt und gesiebt (II). Die Mischungen I und II werden dann gemischt und mit dem gesiebten Magnesiumstearat wird durch einen weiteren Mischprozess die abfüllfertige Pulvermischung hergestellt. Diese Endmischung wird in Hartgelatinekapseln geeigneter Grösse abgefüllt.

Die therapeutische Wirkung von Cilazapril bei Gefässverletzung kann dem nachfolgenden Versuch entnommen werden. Der Versuch ist so beschrieben, dass jedermann, der die nötigen Kenntnisse und Einrichtungen besitzt, ihn nachvollziehen kann.

Eine Arterie wird mit Hilfe eines Ballonkatheters, der durch das Gefässlumen geschoben wird, von innen verletzt. (Ballonisierung). Dabei wird die innerste Zellschicht, das Endothel, entfernt und die Muskelzellen der Media leicht geschädigt. Die Muskelzellen werden dadurch zur Proliferation und Migration in die Intima angeregt. Neues Gewebe, die Neointima, entsteht. (Laboratory Investigation, 49, 327-333, (1983) und Pathologia et Microbiologia, 29, 393-405, (1966)). 14 Tage nach der Ballonisierung wird die Arterie für morphometrische Studien fixiert. Das Ausmass der Neointimabildung wird anschliessend an Querschnitten durch die Arterie mit Hilfe morphometrischer Methoden ausgemessen.

Männliche Ratten, Stamm: RORO (Durchschnittsgewicht 399±6 g; 4-5 Monate alt; Institut für biologischmedizinische Forschung, Füllinsdorf) wurden verwendet. Die Tiere wurden randomisiert in eine Kontrollgruppe und in eine Behandlungsgruppe eingeteilt. Es wurden je 11 Tiere als Kontroll- bzw. behandelte Tiere eingesetzt.

Die Tiere der Behandlungsgruppe erhielten 20 Tage lang soviel Cilazapril dem Futter beigemischt, dass die tägliche Cilazaprilaufnahme durchschnittlich 10 mg/kg betrug. Es handelt sich um eine Dosierung, die den systolischen Blutdruck um ca. 25% senkte. Die Kontrolltiere erhielten das gleiche Laborfutter ohne Zusatz. Die Ballonisierung der Arteria carotis communis wurde 6 Tage nach der ersten Gabe von Cilazapril durchgeführt. Die Tiere wurden mit Aetherinhalation und subcutaner Verabreichung von 200 mg/kg Hexobarbital-Na narkotisiert. Die distale linke Arteria carotis communis und das Bifurkationsgebiet von Carotis externa und interna wurden freigelegt. Der FOGARTY-Katheter (12-060-2F, Edwards Laboratories, Santa Anna, CA, USA) wurde durch die Arteria carotis externa in die Arteria carotis communis bis zum Aortenbogen eingeführt, der Ballon mit Wasser gefüllt (Füllungsvolumen ca. 0,01-0,015 ml) und gegen einen leichten Widerstand zurückgezogen. Dieses Procedere wurde zweimal wiederholt. Die Arteria carotis externa wurde ligiert und die Wunde vernäht. Die Operateure hatten keine Kenntnis, ob es sich um behandelte oder unbehandelte Tiere handelte.

14 Tage nach der Ballonisierung wurden die Ratten mit Aether anaesthesiert und die Carotiden durch Perfusion mit Fixationsmittel (2,5% Glutaraldehyd in 0,1 M Phosphatpuffer, pH 7,4), fixiert. Hierzu wurde eine Sonde durch den linken Herzventrikel in die aufsteigende Aorta geführt (Einfluss) und eine zweite Sonde durch den rechten Ventrikel in den Vorhof geschoben (Abfluss). Das Gefässsystem wurde zunächst mit 10 ml PBS ("phosphate buffered saline"; gepufferte isotonische NaCl-Lösung) gespült und dann 15 min lang mit Fixationsmittel bei einem Druck von 90 mm Quecksilbersäule fixiert. Anschliessend wurden sowohl die linke (ballonisierte) als auch die rechte (nicht-ballonisierte) Arteria carotis (Kontrolle) herauspräpariert, von Gewebe befreit und zur weiteren Fixation in 2,5% Glutaraldehyd in 0,1 M Cacodylat-Puffer eingelegt. Jede Arteria carotis wurde vom distalen zum proximalen Ende in fünf Gefässsegmente aufgeteilt, entwässert und in EPON 812 (eingetragene Marke der Shell A.G.) eingebettet. Das mittlere Segment wurde für die morphologischen Untersuchungen verwendet.

Semidünne Querschnitte (1 μm dick) wurden mit Toluidinblau und basischem Fuchsin gefärbt. Die folgenden Parameter ((1)-(5)) wurden mit dem Morphometrie-System DIASYS (Datalab, Heinz Meyer, CH-

7

3367 Thörigen) gemessen:

(1) Die Querschnittsfläche der Neointima in $\mu m^2$

(2) Die Querschnittsfläche der Media in $\mu m^2$

(3) Die Querschnittsfläche des Lumens in $\mu m^2$

(4) Die Länge der Lamina elastica interna im Querschnitt in $\mu m$

(5) Die Länge der mit Neointima bedeckten Lamina elastica interna in $\mu m$

Aus diesen Messungen wurden folgende Parameter berechnet:

(6) Die mittlere Dicke der Neointima in $\mu m$, berechnet als Kreisring aus der Querschnittsfläche der Neointima. Hierzu wurde der Neointimaquerschnitt in einen Kreisring transformiert, dessen äussere Begrenzung die Lamina elastica interna bildete.

(7) Die Querschnittsfläche der Neointima zur Querschnittsfläche der Media in Prozent (normalisierte Neointimafläche)

(8) Der Prozentsatz der mit Neointima bedeckten Lamina elastica interna.

In der nachstehenden Tabelle sind die wesentlichen morphologischen Charakteristika der Neointima in der ballonisierten Arteria carotis von unbehandelten Tieren und Cilazapril-behandelten Tieren aufgezeigt. Im einzelnen wurden folgende Resultate erhalten:

- Die Querschnittsflächen ($\mu m^2$; Mittelwert ± Standardabweichung) der Neointima (Parameter (1)), waren bei den Kontrolltieren $109 \times 10^3 \pm 51 \times 10^3$ und bei den Cilazapril-behandelten Tieren $20 \times 10^3 \pm 18 \times 10^3$, was einer Hemmung von 82% entspricht.

- Die Querschnittsfläche (Parameter (3); in $\mu m^2$) des Lumens waren bei den Kontrolltieren $324 \times 10^3 \pm 91 \times 10^3$ und bei den Cilazapril-behandelten Tieren $468 \times 10^3 \pm 114 \times 10^3$. Für den Blutfluss steht somit nach Cilazaprilbehandlung eine wesentlich grössere Lumenfläche zur Verfügung.

- Die mittleren Dicken der Neointima (Parameter (6); in $\mu m$) betrugen für Kontrolltiere und Cilazaprilbehandelte Tiere 50±25 und 8±7, was einer Hemmung von 84% entspricht.

- Das Verhältnis der Querschnittsfläche der Neointima zur Querschnittsfläche der Media (Parameter (7)) betrug 101±43% für die Kontrollen und 23±21% für die behandelten Tiere.

- Die Lamina elastica interna von unbehandelten, ballonisierten Tieren waren zu 93±15% mit Neointima bedeckt (Parameter (8)). Bei mit Cilazapril-behandelten Tieren war diese Bedeckung nur noch 35±29%.

Die hemmende Wirkung von Cilazapril auf die Neointimabildung war bei allen Parametern statistisch hochsignifikant. Die Neointimabildung war bei 3 von 11 Tieren vollständig gehemmt. In allen nichtballonisierten Arterien (rechte Arteria carotis) waren keine Gefässveränderungen festzustellen.

Hiermit ist eindeutig gezeigt, dass Cilazapril die durch die arterielle Verletzung hervorgerufene Neointimabildung und deren Konsequenzen unterdrückt.

|  | Unbehandelte Tiere ( n = 11) | Mit Cilazapril behandelte Tiere ( n = 11) |
|---|---|---|
| Querschnittsfläche der Neointima ($\mu m^2$) | $109 \times 10^3 \pm 51 \times 10$ | $20 \times 10^3 \pm 18 \times 10^3$*** |
| Querschnittsfläche des Lumens ($\mu m^2$) | $324 \times 10^3 \pm 91 \times 10^3$ | $468 \times 10^3 \pm 114 \times 10^3$** |
| Mittlere Dicke der Neointima ($\mu m$) | 50 ± 25 | 8 ± 7 *** |
| Verhältnis von Querschnittsfläche der Neointima zu Querschnittsfläche der Media (%) | 101 ± 43 | 23 ± 21 *** |
| Prozentsatz von mit Neointima bedeckter Lamina elastica interna (%) | 93 ± 15 | 35 ± 29 *** |

Bedeutung der Zeichen:

n: Zahl der Versuchstiere;

***: 2p < 0.001 (t-Test)

**: 2p < 0.01 (t-Test)

Chronischer Bluthochdruck führt zu Hypertrophie der Media in den grossen, muskulären Arterien. Erfolgreiche Behandlung des Bluthochdrucks mit ACE-Inhibitoren, sowie in geringerem Masse auch mit ß-Blockern und anderen gängigen Therapeutika wie Vasodilatatoren, führt zu einer Rückbildung der Hypertrophie. (Hypertension, 9, 178-187, (1987)).

Die hier beschriebene, neue Verwendung von ACE-Inhibitoren unterscheidet sich von derjenigen, die zur Rückbildung der Hypertrophie führt, dadurch, dass hier die Bildung der Neointima gehemmt wird. Sie kann sowohl bei Hypertonikern als auch bei Normotonikern angezeigt sein, wenn die oben beschriebenen Prozesse in der Gefässwand eintreten.

Die gegenwärtig am besten untersuchte Gefässverletzung entsteht bei der perkutanen, transluminalen coronaren Angioplastie (PTCA). Hier wird durch Aufblasen eines Ballonkatheters ein stenosiertes oder verschlossenes Gefäss wieder gängig gemacht. (Diese Prozedur ähnelt sehr der oben beschriebenen Ballonisierung). Ein häufig auftretendes Problem ist die sogenannte Restenosierung, d.h. ein Wiederverschliessen. Es ist z.B. bekannt, dass bei coronaren Herzgefässen eine Restenosierung von ca. 30% innerhalb von 6 Monaten auftritt. (The New England Journal of Medicine, 318, 1734-1737, (1988)). Anatomische Studien haben gezeigt, dass Restenosierung bzw. Wiederverschluss am Ort der Verletzung auftritt.

Eine andere klinische Anwendung ist bei arteriösvenösen Transplantationen zu sehen. Restenosierung geschieht z.B. bei sogenannten Bypass-Operationen, wo ein Venen- oder Arterientransplantat als Ersatz für Arterien, beispielsweise Herzkranzgefässen oder Carotiden, eingesetzt wird. Durch Proliferation von glatten Muskelzellen kann ein Verschluss (vor allem an den Nahtstellen) zustandekommen.

Schliesslich spielen Migration und Proliferation von glatten Muskelzellen in einer ganzen Reihe von Kranheiten eine wichtige Rolle. Hier seien insbesondere Arteriosklerose und diabetische Angiopathien genannt. (The New England Journal of Medicine, 314, 488-500, (1986)).

**Patentansprüche**

1. Verwendung von ACE-Inhibitoren zur Herstellung von Mitteln zur Verhinderung der Proliferation glatter Muskelzellen in der Intima und/oder der Media nach Gefässverletzung.

2. Verwendung von ACE-Inhibitoren zur Herstellung von Mitteln zur Verhinderung der Migration glatter Muskelzellen von der Media in die Intima nach Gefässverletzung.

3. Verwendung von ACE-Inhibitoren zur Herstellung von Mitteln zur Verhinderung der Bildung extrazellulärer Matrix nach Gefässverletzung.

4. Verwendung von ACE-Inhibitoren zur Herstellung von Mitteln zur Behandlung und Verhinderung von Neointimabildung nach Gefässverletzung.

5. Verwendung nach einem oder mehreren der Ansprüche 1-4, dadurch gekennzeichnet, dass es sich bei der Gefässverletzung um eine physikalisch, chemisch oder chirurgisch bedingte Verletzung handelt.

6. Verwendung nach Anspruch 5 zur Herstellung von Mitteln zur Verhinderung von arterieller Restenosierung nach Angioplastie.

7. Verwendung nach Anspruch 5 zur Herstellung von Mitteln zur Verhinderung von Gefässwandverdickung nach Gefässchirurgie in Arterien und Venen.

8. Verwendung von ACE-Inhibitoren zur Herstellung von Mitteln zur Prophylaxe von Arteriosklerose und diabetischen Angiopathien

9. Verwendung nach einem der Ansprüche 1-8, dadurch gekennzeichnet, dass es sich bei den ACE-Inhibitoren um Alacepril, Benazepril, Captopril, Cilazapril, Delapril, Enalapril, Enalaprilat, Fosinopril, Lisinopril, Perindopril, Quinapril, Ramipril, Spirapril, Zofenopril und MG 838, vorzugsweise um Cilazapril, handelt.

**Claims**

1. The use of ACE inhibitors for the manufacture of medicaments for the prevention of the proliferation of smooth muscle cells in the intima and/or the media after vascular damage.

2. The use of ACE inhibitors for the manufacture of medicaments for the prevention of the migration of smooth muscle cells from the media into the intima after vascular damage.

3. The use of ACE inhibitors for the manufacture of medicaments for the prevention of the formation of extracellular matrix after vascular damage.

EP 0 363 671 B1

**4.** The use of ACE inhibitors for the manufacture of medicaments for the treatment and prevention of neointima formation after vascular damage.

**5.** The use according to one or more of claims 1-4, characterized in that the vascular damage is damage caused physically, chemically or surgically.

**6.** The use according to claim 5 for the manufacture of medicaments for the prevention of arterial restenosis after angioplasty.

**7.** The use according to claim 5 for the manufacture of medicaments for the prevention of vascular wall thickening in arteries and veins after vascular surgery.

**8.** The use of ACE inhibitors for the manufacture of medicaments for the prophylaxis of arteriosclerosis and diabetic angiopathy.

**9.** The use according to any one of claims 1-8, characterized in that the ACE inhibitors are alacepril, benazepril, captopril, cilazapril, delapril, enalapril, enalaprilat, fosinopril, lisinopril, perindopril, quinapril, ramipril, spirapril, zofenopril and MC 838, preferably cilazapril.

**Revendications**

**1.** Utilisation des inhibiteurs de l'ACE pour la préparation d'agents d'inhibition de la prolifération des cellules musculaires lisses dans les intimas et/ou les médias après des lésions vasculaires.

**2.** Utilisation des inhibiteurs de l'ACE pour la préparation d'agents d'inhibition de la migration des cellules musculaires lisses des médias vers les intimas après des lésions vasculaires.

**3.** Utilisation des inhibiteurs de l'ACE pour la préparation d'agents d'inhibition de la formation d'une matrice extracellulaire après des lésions vasculaires.

**4.** Utilisation des inhibiteurs de l'ACE pour la préparation d'agents pour le traitement et l'inhibition de la formation de néo-intimas après des lésions vasculaires.

**5.** Utilisation selon l'une ou plusieurs des revendications 1 à 4, caractérisée en ce cu'il s'agit, en ce qui concerne la lésion vasculaire, d'une lésion provoquée physiquement, chimiquement ou chirurgicalement.

**6.** Utilisation selon la revendication 5 pour la préparation d'agents pour l'inhibition de la resténose artérielle après angioplastie.

**7.** Utilisation selon la revendication 5 pour la préparation d'agents d'inhibition de l'épaississement des parois des vaisseaux après chirurgie vasculaire dans les artères et les veines.

**8.** Utilisation des inhibiteurs d'ACE pour la préparation d'agents pour la prophylaxie de l'artériosclérose et des angiopathies diabétiques.

**9.** Utilisation selon l'une quelconque des revendications 1 à 8, caractérisée en ce qu'il s'agit, en ce qui concerne les inhibiteurs d'ACE, de: Alacepril, Bénazépril, Captopril, Cilazapril, Delapril, Enalapril, Enalaprilate, Fosinopril, Lisinopril, Périndopril, Quinapril, Ramipril, Spirapril, Zofénopril et MC 838, de préférence de Cilazapril.